# EUROPEAN PATENT APPLICATION

(11) **EP 3 572 069 A1**
(43) Date of publication of application: **27.11.2019**
(21) Application number: 19020336.4
(22) Date of filing: 20.05.2019
(51) Int. Cl.: A61K 9/00, A61K 9/14, A61K 9/16, A61K 31/4166, A61K 9/20

(54) **INCREASING SOLUBILITY AND BIOAVAILABILITY OF ENZALUTAMIDE**

(30) Priority: 21.05.2018 CZ 20180234
(71) Applicant: Zentiva K.S., 102 37 Praha 10 (CZ)
(72) Inventor: Obadalova, Iva, 169 00 Praha 6 (CZ); Schongut, Marek, 140 00 Praha 4 - Podoli (CZ); Krejcik, Lukas, 190 17 Praha - Vinor (CZ); Zvatora, Pavel, 198 00 Praha 9 - Cerny Most (CZ)
(74) Representative: Jirotkova, Ivana

(57) **Abstract**

The submitted solution relates to the pharmaceutical mixture containing amorphous enzalutamide and at least one excipient from the group of C₅ to C₁₂ saccharides or their derivatives or their derivatives with possible another substance, wherein the mixture is in the form of solid premix.

## Description

### Field of the Invention

Enzalutamide is a substance of high permeability; the only parameter limiting its bioavailability is its very poor solubility. Currently, enzalutamide is available on the market just in the form of soft gelatine capsules containing solution of enzalutamide in an oil-based medium. Crystalline enzalutamide is a very poorly soluble substance. Amorphous form of enzalutamide dissolves better, however, the solution becomes oversaturated very quickly and, subsequently, enzalutamide precipitates in the crystalline form. The present invention describes using of the amorphous form of enzalutamide in the solid medicine form together with excipients that influence enzalutamide behaviour during dissolving and can significantly suppress its re-precipitation to crystalline enzalutamide.

### Background Art

Enzalutamide, MDV3100, chemically 4-(3-(4-cyano-3-(trifluoromethyl)phenyl)-5,5-dimethyl-4-oxo-2-thioxo-1-imidazolidinyl)-2-fluoro-N-methyl-benzamide, is a drug intended for therapy of patients with prostatic carcinoma who suffer from progression following the androgen deprivation therapy (castration-resistant prostatic carcinoma - CRPC). This is a targeted inhibitor of androgen receptor (AR) which is competitively linked to its ligand-binding C-terminal domain and disturbs translocation of AR into the core, intake of AR cofactors, and linkage of AR to DNA.

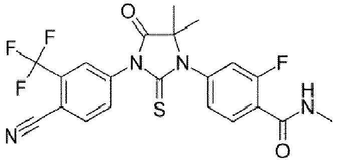

The enzalutamide molecule was, together with similar substances, first mentioned in international patent application WO 2006/124118. Here, enzalutamide was isolated in the form of colourless crystalline substance and its pharmacological effects as well as its synthesis were described.

Three crystalline forms of enzalutamide itself are known. Patent application WO 2014/041487 discloses crystalline forms R1 and R2. Patent application CZ2016-275 discloses another crystalline form designated as form 5. Form R1 is the most stable crystalline form, crystalline forms R2 and 5 are, regarding physical stability, less stable (metastable) and, during thermal analysis, for instance, both these forms recrystallize to the most stable crystalline form R1. Enzalutamide belongs among very low soluble substances. The amorphous form of enzalutamide shows better solubility; it dissolves better, however, its solution becomes very quickly oversaturated and crystalline form R1 subsequently precipitates. The objective of this invention, therefore, was to prepare such pharmaceutical mixtures of amorphous enzalutamide that would suppress its subsequent precipitation, manage to keep the dissolved enzalutamide in solution in the oversaturated state, and, at the same time, be chemically and physically stable. Some solutions running this way are indicated in patent applications WO 2014/043208 and WO 2015/118015. The former application describes preparation of solid solutions with polymers and the latter discloses deposition of enzalutamide onto porous silicon dioxide in connection with a wetting agent. Using of synthetic polymers as well as solid carriers, including non-toxic nanoparticles or microparticles, microcrystalline cellulose, silicon dioxide, aluminium oxide and non-toxic wetting agent in the composition, is already mentioned in basic patent WO 2006/124118.

In application WO 2014/043208, the most preferred polymer seems to be HPMCAS. According to the cited application, the solid solution with this polymer was developed for reducing the number of enzalutamide doses. Whereas in the case of currently administered form of the solution in soft gelatine capsules, 4 doses, 40 mg each, are necessary, the objective of development in the cited application was to administer just one 160 mg tablet with the same effect. Example 15, however, shows that the newly developed composition does not reach full biological availability of the so far administered doses. Therefore, it is still worth working on new types of compositions that would increase biological availability of enzalutamide.

### Disclosure of the Invention

The subject of this invention is pharmaceutical mixtures of amorphous enzalutamide and excipient from the group of C₅ to C₁₂ saccharides or from the group of derivatives derived from C₅ to C₁₂ saccharides, and their using in medicine forms. Such mixtures can show good solubility without subsequent precipitation, as well as physical and chemical stability. In some cases, solubility of the mixture can be enhanced by addition of a wetting agent.

Pharmaceutical mixtures which are the subject of this invention are in the form of solid premix. In the pharmaceutical composition, particles of individual components of the solid premix are in close vicinity. The solid premix can be prepared, for instance, by dissolving or stirring the mixture components in a solvent or mixture of solvents and subsequent evaporation of the solvent or mixture of solvents by, for instance, spray drying, freeze-drying, or evaporation in a rotary vacuum evaporator. The solid premix can also be prepared by melting well homogenized mixture of individual components of the premix.

Good solvents for preparing pharmaceutical mixtures of this invention have proved to be alcohols, particularly lower C1 to C4 alcohols, for instance, methanol or ethanol; or ketones, particularly C3 to C6 ketones, for instance, acetone or ethyl-methyl ketone.

Temperature of melting is given by melting points of both components of the pharmaceutical mixture, for instance, 190 to 250°C.

In one embodiment, the pharmaceutical mixture is amorphous enzalutamide and pharmaceutically acceptable saccharide or its derivative. The group of suitable saccharides or their derivatives includes, for instance, mannitol, sorbitol, maltitol, sucrose, etc. In the preferred embodiment, the saccharide is mannitol. Weight ratio of saccharide or its derivative in this pharmaceutical mixture can be within the range of 30% to 90%. In the preferred embodiment, weight ratio of saccharide or its derivative in this pharmaceutical mixture is within the range of 50% to 85%.

The pharmaceutical mixture consisting of amorphous enzalutamide and mannitol is characterized by the differential scanning calorimetry curve showing glass transition temperature of enzalutamide of 92°C, subsequent crystallization of the amorphous form at 132°C to crystalline form R1 with melting temperature of 192°C; the DSC curve also shows melting temperature of mannitol 162°C.

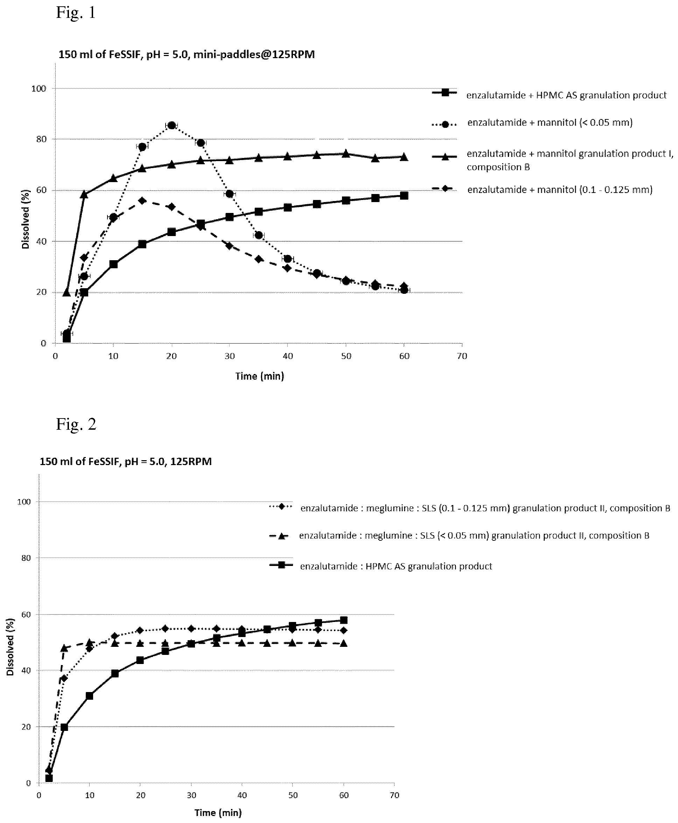

Solubilities of the mixtures of amorphous enzalutamide with mannitol, the granulation product containing the mixture of amorphous enzalutamide with mannitol, and the granulation product containing amorphous enzalutamide and HPMC AS are compared in **Fig. 1****.** The mixture of amorphous enzalutamide and mannitol was prepared by melting and, subsequently, the material was crushed and screened to fractions of particles under 0.05 mm and 0.1 to 0.125 mm. These two materials were tested by dissolution. The mixture with fraction of particles under 0.05 mm showed quick dissolving and subsequent quick precipitation, whereas the mixture with fraction of particles 0.1 to 0.125 mm snowed slower dissolving and also subsequent slower precipitation. After dry granulation of the mixture of amorphous enzalutamide with mannitol with fraction of particles 0.1 to 0.125 mm, this process supported solubility of the material and suppressed precipitation. In comparison with the granulation product containing the mixture of amorphous enzalutamide and HPMC AS, the granulation product prepared from the mixture of amorphous enzalutamide and mannitol showed higher solubility.

In another embodiment, the pharmaceutical mixture contains amorphous enzalutamide, meglumine and sodium lauryl sulphate (SLS). Weight ratio of meglumine in this pharmaceutical mixture can be within the range of 30% to 90%. In the preferred embodiment, weight ratio of meglumine in this pharmaceutical mixture is within the range of 50% to 85%, preferably 65% to 75%. Content of SLS in this pharmaceutical mixture can be within the range of ratios of enzalutamide : SLS 1 : 0.025 to 1 : 0.3. In the preferred embodiment, content of SLS in this pharmaceutical mixture is in the ratio of enzalutamide : SLS 1 : 0.1. The pharmaceutical mixture consisting of amorphous enzalutamide, meglumine and SLS is characterized by the differential scanning calorimetry curve showing glass transition temperature of enzalutamide of 93°C without subsequent crystallization of the amorphous form to crystalline form R1; the curve also shows melting temperature of meglumine 125°C. The fact that, during the thermal analysis, recrystallization of the amorphous form to the crystalline form vanishes in the mixture of enzalutamide and meglumine implies that this pharmaceutical mixture will be physically more stable than the mixture where this recrystallization takes place during the thermal analysis.

Solubilities of the granulation products containing the mixture of amorphous enzalutamide with meglumine are indicated in **Fig. 2****.** Experiments have proved that suitable amount of SLS to be added for optimum dissolving of amorphous enzalutamide is 5% to 15% by weight, preferably 10% by weight of SLS, relatively to enzalutamide. Following the preparation of the pharmaceutical mixture of enzalutamide, meglumine and SLS, the material was screened and two granulation products were prepared from the fractions of particles under 0.05 mm and 0.1 to 0.125 mm for subsequent testing. The kinetics of dissolving implies that the granulation product containing the pharmaceutical mixture of smaller particle size (< 0.05 mm) was first dissolved more quickly than the granulation product containing the pharmaceutical mixture of larger particle size (0.1 to 0.125 mm).

In another embodiment, the pharmaceutical mixture contains amorphous enzalutamide and pharmaceutically acceptable substituted derivative of C₅ to C₇ carboxylic acid. Suitable substituted derivatives of C₅ to C₇ carboxylic acid include, for instance, ascorbic acid, sorbic acid, citric acid, adipic acid, etc. In the preferred embodiment, the substituted derivative of C₅ to C₇ carboxylic acid is sorbic acid or ascorbic acid. Weight ratio of the substituted derivative of C₅ to C₇ carboxylic acid in this pharmaceutical mixture can be within the range of 30% to 90%. In the preferred embodiment, weight ratio of the substituted derivative of C₅ to C₇ carboxylic acid in this pharmaceutical mixture is within the range of 50% to 85%, preferably 65% to 75%.

The pharmaceutical mixture consisting of amorphous enzalutamide and ascorbic acid is characterized by the differential scanning calorimetry curve showing glass transition temperature of enzalutamide of 84°C, subsequent crystallization of the amorphous form at 142°C to crystalline form R1 with melting temperature of 190°C; the curve also shows melting temperature of ascorbic acid of 181°C.

The pharmaceutical mixture consisting of amorphous enzalutamide and sorbic acid is characterized by the differential scanning calorimetry curve showing glass transition temperature of enzalutamide of 74°C, subsequent crystallization of the amorphous form at 100°C to crystalline form R1 with melting temperature of 192°C; the curve also shows melting temperature of sorbic acid of 124°C.

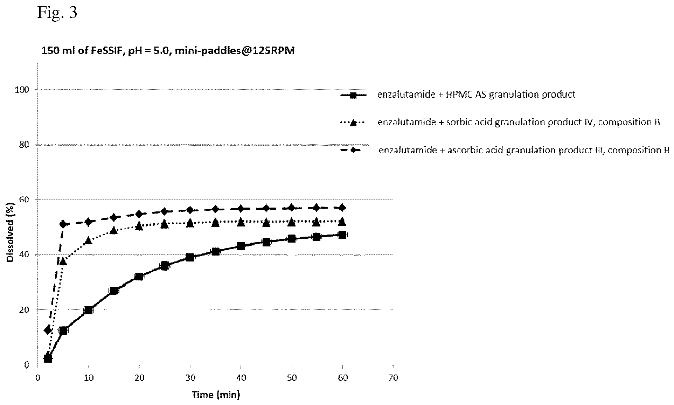

Solubilities of the granulation products containing the mixture of amorphous enzalutamide with ascorbic and sorbic acids are indicated in Fig. 3. In comparison with the granulation product containing the mixture of amorphous enzalutamide and HPMC AS, the granulation products containing amorphous enzalutamide and ascorbic or sorbic acid showed higher solubility.

Another subject of the invention is the pharmaceutical composition containing some of above mentioned pharmaceutical mixtures in the form of the above defined premix and at least one pharmaceutically acceptable excipient. Particularly preferred pharmaceutical mixture for using in the pharmaceutical formulation is the pharmaceutical mixture of amorphous enzalutamide and mannitol in weight ratio of enzalutamide : mannitol 1 : 3, the pharmaceutical mixture of amorphous enzalutamide, meglumine and sodium lauryl sulphate (SLS) in weight ratio of enzalutamide : meglumine : SLS 1 : 3 : 0.1, the pharmaceutical mixture of amorphous enzalutamide and ascorbic acid in weight ratio of enzalutamide : ascorbic acid 1 : 3, and the pharmaceutical mixture of amorphous enzalutamide and sorbic acid in weight ratio of enzalutamide : sorbic acid 1 : 3. Content of enzalutamide in the pharmaceutical composition is 40 mg, 80 mg or 160 mg.

The composition also includes one or more pharmaceutically acceptable excipients that serve mainly as fillers, binders, lubricants, surfactants, disintegrators, colourants, solvents, antimicrobial agents, or flavour and olfactory corrigents. Preferably, the composition includes at least one excipient selected from the group comprising lactose, microcrystalline cellulose, sodium carboxymethyl starch, silicon dioxide, magnesium stearate, and their combinations. The pharmaceutical composition can be prepared in virtually any solid medicine form, e.g. in the form of tablets, capsules, powders, pellets or granules. Preferred medicine form is capsule, tablet or coated tablet. Preferably, the capsule can be prepared in such a way that the pharmaceutical mixture of amorphous enzalutamide is mixed with pharmaceutically acceptable excipients and filled into capsules following possible previous granulation. Preferably, the tablet can be prepared in such a way that the pharmaceutical mixture - premix of amorphous enzalutamide is mixed with pharmaceutically acceptable excipients and tableted, with possible subsequent coating.

### Brief description of the Drawings

- Fig. 1:: Dissolution behaviour of the mixture of amorphous enzalutamide with mannitol.
- Fig. 2:: Dissolution behaviour of the mixture of amorphous enzalutamide with meglumine, and
- Fig. 3:: Dissolution behaviour of the mixture of amorphous enzalutamide with ascorbic acid and with sorbic acid.

### Examples

The following examples of the embodiment serve just for illustration and explanation of the invention, and in no case are they intended as limiting the scope of protection, as defined just by reading of the claims.

Crystalline form R1 of enzalutamide was prepared by synthesis disclosed in patent application WO 2006/124118.

### List of excipients used in the following examples:

Mannitol - Parteck M200
Meglumine
Sodium lauryl sulphate
Ascorbic acid
Sorbic acid
Sodium croscarmellose - Ac-Di-Sol
Microcrystalline cellulose - Avicel PH-102
Lactose - Lactose monohydrate
Silicon dioxide - Aerosil A-200
Magnesium stearate

### Example 1

### Preparation of pharmaceutical mixture of amorphous enzalutamide and Parteck M200

1 g of enzalutamide of crystalline form R1 and 3 g of mannitol were weighed. This mixture was dissolved in 100 ml of methanol in an ultrasound bath and then evaporated in a rotary vacuum evaporator. The solid product was screened through a 0.5 mm sieve and subsequently dried in a vacuum drying oven at 10 kPa for 24 hours. After drying, the material was micronized in a jet mill at pressure of 500 kPa.

### Example 2

### Preparation of pharmaceutical mixture of amorphous enzalutamide and Parteck M200

6 g of enzalutamide of crystalline form R1 and 18 g of mannitol were weighed. This mixture was screened through a 0.5 mm sieve and subsequently stirred in Turbula at 46 r.p.m. for 30 min. The intermixed mixture was extruded in a twin-screw extruder ThreeTec at temperature of 220°C. The extrudate was then comminuted, screened through a 0.5 mm sieve, and micronized in a jet mill at pressure of 500 kPa.

### Example 3

### Preparation of pharmaceutical mixture of amorphous enzalutamide, meglumine and SLS

2 g of enzalutamide of crystalline form R1, 6 g of meglumine, and 0.2 g of sodium lauryl sulphate were weighed. This mixture was dissolved in 200 ml of methanol in an ultrasound bath and then evaporated in a rotary vacuum evaporator. The solid product was screened through a 0.5 mm sieve and subsequently dried in a vacuum drying oven at 10 kPa for 24 hours.

### Example 4

### Preparation of pharmaceutical mixture of amorphous enzalutamide, meglumine and SLS

6 g of enzalutamide of crystalline form R1, 18 g of meglumine, and 0.6 g of sodium lauryl sulphate were weighed. This mixture was dissolved in 700 ml of methanol in an ultrasound bath at temperature of 40°C during 30 min and subsequently sprayed in a spray drying oven Buchi B-290 at temperature of 85°C at the inlet. The solid product was then dried in a vacuum drying oven at 10 kPa for 24 hours.

### Example 5

### Preparation of pharmaceutical mixture of amorphous enzalutamide and ascorbic acid

1.5 g of enzalutamide of crystalline form R1 and 4.5 g of ascorbic acid were weighed. This mixture was dissolved in 100 ml of methanol in an ultrasound bath and then evaporated in a rotary vacuum evaporator. The solid product was screened through a 0.5 mm sieve and subsequently dried in a vacuum drying oven at 10 kPa for 24 hours. After drying, the material was micronized in a jet mill at pressure of 500 kPa.

### Example 6

### Preparation of pharmaceutical mixture of amorphous enzalutamide and ascorbic acid

12 g of enzalutamide of crystalline form R1, 36 g of ascorbic acid were weighed. This mixture was dissolved in 850 ml of methanol in an ultrasound bath and subsequently sprayed in a spray drying oven Buchi B-290 at temperature of 85°C at the inlet. The solid product was then dried in a vacuum drying oven at 10 kPa for 24 hours.

### Example 7

### Preparation of pharmaceutical mixture of amorphous enzalutamide and sorbic acid

1 g of enzalutamide of crystalline form R1 and 3 g of sorbic acid were weighed. This mixture was dissolved in 60 ml of methanol in an ultrasound bath and then evaporated in a rotary vacuum evaporator. The solid product was screened through a 0.5 mm sieve and subsequently dried in a vacuum drying oven at 10 kPa for 24 hours. After drying, the material was micronized in a jet mill at pressure of 500 kPa.

### Example 8

### Preparation of pharmaceutical mixture of amorphous enzalutamide and sorbic acid

12 g of enzalutamide of crystalline form R1, 36 g of sorbic acid were weighed. This mixture was dissolved in 700 ml of methanol in an ultrasound bath and subsequently sprayed in a spray drying oven Buchi B-290 at temperature of 90°C at the inlet. The solid product was then dried in a vacuum drying oven at 10 kPa for 24 hours.

### Example 9

### Composition of tested granulation products

The prepared pharmaceutical mixture was mixed with sodium croscarmellose, microcrystalline cellulose, lactose, and silicon dioxide according to the following table. The obtained mixture was screened through a 0.6 mm sieve and subsequently homogenized in Turbula at 49 r.p.m. for 10 min. After addition of magnesium stearate, the mixture was homogenized in Turbula at 49 r.p.m. for additional 3 min. The granulation product was obtained by tableting this homogenized mixture and subsequent crushing on a 0.8 mm sieve. The tables show examples of granulation products of three different compositions. Compositions A and B are the compositions (formulations) containing lactose. Composition C is the tested composition (formulation) without lactose. The dissolution experiments have proved that composition of the formulation has no significant effect on the course of dissolving. The granulation product I contains the pharmaceutical mixture of amorphous enzalutamide and Parteck M200. The granulation product II contains the pharmaceutical mixture of amorphous enzalutamide, meglumine, and SLS. The granulation product III contains pharmaceutical mixture of amorphous enzalutamide and ascorbic acid. The granulation product IV contains the pharmaceutical mixture of amorphous enzalutamide and sorbic acid.

| **Composition A** | Granulation product I | Granulation product II | Granulation product III | Granulation product IV |
|---|---|---|---|---|
| enzalutamide | 13.3% | 13.3% | 13.3% | 13.3% |
| Parteck M200 | 40% | - | - | - |
| Meglumine | - | 40% | - | - |
| SLS | - | 1.3% | - | - |
| Ascorbic acid | - | - | 40% | - |
| Sorbic acid | - | - | - | 40% |
| Sodium croscarmellose | 8.0% | 8.0% | 8.0% | 8.0% |
| Microcrystalline cellulose | 19.0% | 19.0% | 19.0% | 19.0% |
| Lactose | 18.9% | 17.6% | 18.9% | 18.9% |
| Silicon dioxide | 0.5% | 0.5% | 0.5% | 0.5% |
| Magnesium stearate | 0.3% | 0.3% | 0.3% | 0.3% |

| **Composition B** | Granulation product I | Granulation product II | Granulation product III | Granulation product IV |
|---|---|---|---|---|
| enzalutamide | 15.0% | 15.0% | 15.0% | 15.0% |
| Parteck M200 | 45.0% | - | - | - |
| Meglumine | - | 45.0% | - | - |
| SLS | - | 1.5% | - | - |
| Ascorbic acid | - | - | 45.0% | - |
| Sorbic acid | - | - | - | 45.0% |
| Sodium croscarmellose | 6.0% | 6.0% | 6.0% | 6.0% |
| Microcrystalline cellulose | 13.5% | 13.5% | 13.5% | 13.5% |
| Lactose | 15.0% | 13.5% | 15.0% | 15.0% |
| Silicon dioxide | 3.5% | 3.5% | 3.5% | 3.5% |
| Magnesium stearate | 2% | 2% | 2% | 2% |

| **Composition C** | Granulation product I | Granulation product II | Granulation product III | Granulation product IV |
|---|---|---|---|---|
| enzalutamide | 17.6% | 17.6% | 17.6% | 17.6% |
| Parteck M200 | 52.8% | - | - | - |
| Meglumine | - | 52.8% | - | - |
| SLS | - | 1.8% | - | - |
| Ascorbic acid | - | - | 52.8% | - |
| Sorbic acid | - | - | - | 52.8% |
| Sodium croscarmellose | 7.5% | 7.5% | 7.5% | 7.5% |
| Microcrystalline cellulose | 17.6% | 15.8% | 17.6% | 17.6% |
| Silicon dioxide | 3.5% | 3.5% | 3.5% | 3.5% |
| Magnesium stearate | 1.0% | 1.0% | 1.0% | 1.0% |

### Example 10

### Preparation of granulation product from pharmaceutical mixture of amorphous enzalutamide and HPMC AS

The pharmaceutical mixture of amorphous enzalutamide and HPMC AS was prepared according to the procedure disclosed in patent application WO 2014/043208. 20 g of enzalutamide of crystalline form R1 and 13.3 g of HPMC AS were weighed. This mixture was dissolved in 750 ml of acetone at 45°C and subsequently sprayed in a spray drying oven Buchi B-290 at temperature of 85°C at the inlet. The solid product was then dried in a vacuum drying oven at 10 kPa for 24 hours. The dried product was then mixed with pharmaceutical excipients according to ratios disclosed in patent application WO 2014/043208, tableted, and crushed on a 0.8 mm sieve. The obtained granulation product was analyzed by dissolution.

| **Composition HPMC AS** | Granulation product |
|---|---|
| enzalutamide | 32.0% |
| HPMC AS | 21.3% |
| Sodium croscarmellose | 7.9% |
| Microcrystalline cellulose | 19.0% |
| Lactose | 19.0% |
| Silicon dioxide | 0.5% |
| Magnesium stearate | 0.3% |

### List of analytical methods

***The differential scanning calorimetric (DSC) measurements*** were carried out using an instrument Discovery DSC of the firm TA Instruments. Weight of the sample in a standard Al pan (40 µL) was 3 - 5 mg and heating rate 5°C / min. The temperature program used includes 5 min of stabilization at temperature of 0°C and heating to 250°C at heating rate of 5°C / min (amplitude = 0.8°C and period = 60 s). 5.0 N₂ of flow rate 50 ml/ min was used as carrier gas.

## Claims

1. Pharmaceutical mixture **characterized in that** it contains amorphous enzalutamide and at least one excipient from the group of C₅ to C₁₂ saccharides or their derivatives or their derivatives with possible another substance, wherein the mixture is in the form of solid premix.

2. Pharmaceutical mixture according to claim 1, **characterized in that** the excipient from the group of C₅ to C₁₂ saccharides or their derivatives can be pharmaceutically acceptable saccharide, preferably mannitol, maltitol, sorbitol or sucrose.

3. Pharmaceutical mixture according to claim 1, **characterized in that** the excipient from the group of C₅ to C₁₂ saccharides or their derivatives can be meglumine together with sodium lauryl sulphate.

4. Pharmaceutical mixture according to claim 1, **characterized in that** the excipient from the group of C₅ to C₁₂ saccharides or their derivatives can be substituted derivative of C₅ to C₇ carboxylic acid, preferably ascorbic acid or sorbic acid.

5. Pharmaceutical mixture according to any of the previous claims, **characterized in that** the weight ratio of excipient from the group of C₅ to C₁₂ saccharides or their derivatives is 30% to 90%, preferably 50% to 85%.

6. Pharmaceutical mixture according to any of the previous claims, **characterized in that** size of minimum 50% of particles of this pharmaceutical mixture (d50) is within the range of 5 to 200 µm, preferably 5 to 125 µm.

7. Pharmaceutical composition, **characterized in that** it contains the pharmaceutical mixture defined in claims 1 to 6 and at least one excipient selected from the group comprising fillers, binders, wetting agents, or lubricants.

8. Pharmaceutical composition according to claim 7 **characterized in that** at least one pharmaceutically acceptable excipient is selected from the group consisting of mannitol, sorbitol, maltitol, sucrose, meglumine together with sodium lauryl sulphate, ascorbic acid, sorbic acid.
